# EUROPEAN PATENT APPLICATION

(11) **EP 1 840 221 A1**
(43) Date of publication of application: **03.10.2007**
(21) Application number: 07006508.1
(22) Date of filing: 29.03.2007
(51) Int. Cl.: C12Q 1/48, G01N 33/573

(54) **Method for measuring kinase activity**

(30) Priority: 31.03.2006 JP 2006101090; 30.06.2006 JP 2006180724
(71) Applicant: SYSMEX CORPORATION, Kobe-shi, Hyogo 651-0073 (JP)
(72) Inventor: Notoya, Michitaka, Kobe-shi, Hyogo 651-0073 (JP); Ohyama, Tomoko, Kobe-shi, Hyogo 651-0073 (JP); Tamura, Shigeyuki, Kobe-shi, Hyogo 651-0073 (JP); Yoshida, Tomokazu, Kobe-shi, Hyogo 651-0073 (JP); Ishihara, Hideki, Kobe-shi, Hyogo 651-0073 (JP)
(74) Representative: HOFFMANN EITLE

(57) **Abstract**

A method for measuring the activity of a receptor kinase present in a cell membrane of a cell is described. The method comprises steps of preparing a sample containing the receptor kinase by separating a cytoplasm from the cell, contacting the receptor kinase in the sample with a substrate to phosphorylate the substrate with the activity of the receptor kinase, binding a labeling substance to the phosphorylated substrate, and measuring the activity of the receptor kinase based on a result of detection of the labeling substance bound to the phosphorylated substrate.

SEQUENCE LISTING

<110> Sysmex corporation

<120> Method for Measuring Kinase Activity

<130> 1-2006-013EP

<160> 7

<170> PatentIn version 3.1

<210> 1
<211> 34
<212> DNA
<213> Artificial sequence

<220>
<223> Designed DNA based on Grb2 gene

<400> 1
cgcggatccc atatggaagc catcgccaaa tatg 34

<210> 2
<211> 27
<212> DNA
<213> Artificial sequence

<220>
<223> Designed DNA based on Grb2 gene

<400> 2
cccaagcttt tagacgttcc ggttcac 27

<210> 3
<211> 25
<212> PRT
<213> Artificial sequence

<220>
<223> Amino acid sequence of poly(Glu,Tyr) peputide

<400> 3

Glu Glu Glu Glu Tyr Glu Glu Glu Glu Tyr Glu Glu Glu Glu Tyr Glu
1               5                   10                  15

Glu Glu Glu Tyr Glu Glu Glu Glu Tyr
            20                  25

<210> 4
<211> 75
<212> DNA
<213> Artificial sequence

<220>
<223> Designed DNA based on amino acid sequence of poly(Glu,Tyr) peptid
e

<400> 4
gaggaggagg agtacgaaga agaagaatat gaagaagaag aatatgaaga agaagaatat 60

gaggaggaag agtac 75

<210> 5
<211> 25
<212> DNA
<213> Artificial sequence

<220>

<223>  Designed DNA based on nucleotide sequence of poly(Glu,Tyr) peptid
      e

<400>  5
ccggggatcc gaggaggagg agtac                                          25


<210>  6
<211>  27
<212>  DNA
<213>  Artificial sequence

<220>
<223>  Designed DNA based on nucleotide sequence of poly(Glu,Tyr) peptid
      e

<400>  6
cccggggaat tcgtactctt cttcctc                                        27


<210>  7
<211>  263
<212>  PRT
<213>  Artificial sequence

<220>
<223>  Amino acid sequence of GST-poly(Glu,Tyr) fusion protein

<400>  7

Met Ser Pro Ile Leu Gly Tyr Trp Lys Ile Lys Gly Leu Val Gln Pro
1               5                   10                  15


Thr Arg Leu Leu Leu Glu Tyr Leu Glu Glu Lys Tyr Glu Glu His Leu
            20                  25                  30


Tyr Glu Arg Asp Glu Gly Asp Lys Trp Arg Asn Lys Lys Phe Glu Leu
        35                  40                  45


Gly Leu Glu Phe Pro Asn Leu Pro Tyr Tyr Ile Asp Gly Asp Val Lys
    50                  55                  60


Leu Thr Gln Ser Met Ala Ile Ile Arg Tyr Ile Ala Asp Lys His Asn
65                  70                  75                  80


Met Leu Gly Gly Cys Pro Lys Glu Arg Ala Glu Ile Ser Met Leu Glu
                85                  90                  95


Gly Ala Val Leu Asp Ile Arg Tyr Gly Val Ser Arg Ile Ala Tyr Ser
            100                 105                 110


Lys Asp Phe Glu Thr Leu Lys Val Asp Phe Leu Ser Lys Leu Pro Glu
        115                 120                 125


Met Leu Lys Met Phe Glu Asp Arg Leu Cys His Lys Thr Tyr Leu Asn
    130                 135                 140


Gly Asp His Val Thr His Pro Asp Phe Met Leu Tyr Asp Ala Leu Asp
145                 150                 155                 160


Val Val Leu Tyr Met Asp Pro Met Cys Leu Asp Ala Phe Pro Lys Leu
                165                 170                 175


Val Cys Phe Lys Lys Arg Ile Glu Ala Ile Pro Gln Ile Asp Lys Tyr

            180                 185                 190
Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Val Pro Arg
    210                 215                 220
Gly Ser Glu Glu Glu Glu Tyr Glu Glu Glu Glu Tyr Glu Glu Glu Glu
225                 230                 235                 240
Tyr Glu Glu Glu Glu Tyr Glu Glu Glu Glu Tyr Glu Phe Pro Gly Arg
                245                 250                 255
Leu Glu Arg Pro His Arg Asp
            260

## Description

### BACKGROUND OF THE INVENTION

### 1. Field of the Invention

The present invention relates to a method of measuring the receptor kinase activity and a reagent kit for measuring the receptor kinase activity.

### 2. Description of the Related Art

It is known that many of receptor kinases present in a cell membrane are associated with malignant alteration of a cell, and the activity of a receptor kinase becomes high (or low) in a malignant cell. For example, It is known that the activity of HER1 belonging to a human epithelial growth factor receptor (hereinafter, referred to as HER) family is high mainly in a lung cancer. It is known that the activity of HER2 belonging to the same family is high mainly in a breast cancer. A receptor kinase such as HER1 and HER2 is activated and autophosphorylated by binding with a ligand, and thereby, can phosphorylate a substrate finally. When a substrate is phosphorylated, a signal is transmitted to a particular protein in a downstream signal transmission system, and thereby, cell proliferation, cell death and the like are controlled. For example, when abnormality occurs in a signal transmission system involved in cell proliferation, abnormal proliferation of a cell may be caused and malignant alteration of a cell may be leaded in some cases. Therefore, measurement of the kinase activity becomes an index for determining malignant alteration of a cell.

As a method of measuring the receptor kinase activity, the method described, for example, in the reference of Knutson et al. (Victoria P Knutson and Ruth Ann Buck (1991) Archives of Biochemistry and Biophysics 285(2): 197-204) is known. According to the method, autophosphorylation of an insulin receptor which is one of receptor kinases can be detected. Specifically, after insulin and a cultured cell are incubated, an insulin receptor is obtained from a cell membrane of this cultured cell, and autophosphorylation is detected using an anti-phosphotyrosine antibody labeled with a radioactive substance. However, by the technique described in the reference of Knutson et al., whether a substrate is phosphorylated with the enzyme activity of an activated receptor kinase or not can not be determined. Therefore, for measuring the activity of the receptor kinase, development of the technique which can detect phosphorylation of a substrate is desired.

### SUMMARY OF THE INVENTION

The scope of the present invention is defined solely by the appended claims, and is not affected to any degree by the statements within this summary.

An object of the present invention is to provide a method and a reagent kit which can detect phosphorylation of a substrate caused by the enzyme activity of an activate receptor kinase.

A first aspect of the present invention relates to a method for measuring the activity of a receptor kinase present in a cell membrane of a cell, comprising steps of:
preparing a sample containing the receptor kinase by separating a cytoplasm from the cell;
contacting the receptor kinase in the sample with a substrate to phosphorylate the substrate by the activity of the receptor kinase;
binding a labeling substance to the phosphorylated substrate; and
measuring the activity of the receptor kinase based on a result of detection of the labeling substance bound to the phosphorylated substrate.

A second aspect of the present invention relates to a method for estimating influence of a receptor kinase inhibitor on a cell, comprising steps of:
measuring the activity of a receptor kinase by the method according to claim 1 to obtain a first measurement result;
measuring the activity of the receptor kinase contacted with the inhibitor by the method according to claim 1 to obtain a second measurement result; and
estimating influence of the inhibitor on the cell based on the first and second measurement results.

A third aspect of the present invention relates to a method for estimating influence of a receptor kinase ligand on a cell, comprising steps of:
measuring the activity of a receptor kinase by the method according to claim 1 to obtain a first measurement result;
measuring the activity of the receptor kinase contacted with the ligand by the method according to claim 1 to obtain a second measurement result; and
estimating influence of the ligand on the cell based on the first and second measurement results.

A fourth aspect of the present invention relates to a reagent kit for measuring the activity of a receptor kinase present in a cell membrane of a cell, comprising:
a substrate for the receptor kinase;
a phosphate group donor containing a phosphate group which is capable of being introduced into the substrate by the activity of the receptor kinase; and
a labeling substance capable of binding to the substrate with a phosphate group introduced therein.

### BRIEF DESCRIPTION OF THE DRAWINGS

Fig. 1 is a fluorescence photograph showing the result of Western blotting in Example 1.
Fig. 2 is a fluorescence photograph showing the result of Slot blot in Example 2.
Fig. 3 is a fluorescence photograph showing the result of Western blotting in Example 3.
Fig. 4 is a fluorescence photograph showing the result of Western blotting in Example 5.
Fig. 5 is a fluorescence photograph showing the result of Western blotting in Example 6.
Fig. 6 is a graph showing the result of Western blotting in Example 6.
Fig. 7 is a graph showing the result of ELISA in Example 7.
Fig. 8 is a graph showing the result of Western blotting in Example 8.
Fig. 9 is a graph showing the result of Western blotting in Example 8.
Fig. 10 is a graph showing the result of Western blotting in Example 8.
Fig. 11 is a graph showing the result of Western blotting in Example 9.

### DESCRIPTION OF THE PREFERRED EMBODIMENTS

In the method of the present embodiment, the activity of a receptor kinase is measured by detecting phosphorylation of a substrate caused by the enzyme activity of a receptor kinase obtained from a cell. Specifically, the method is a method of measuring the activity of a receptor kinase present in a cell membrane of a cell, and comprises the following steps:
preparing a sample containing the receptor kinase by separating a cytoplasm from the cell,
contacting the receptor kinase in the sample with a corresponding substrate thereto to phosphorylate the substrate by the activity of the receptor kinase,
binding a detectable labeling substance to the phosphorylated substrate, and
measuring the activity of the receptor kinase based on the result of detection of the labeling substance bound to the phosphorylated substrate.
According to this method, a substrate phosphorylated by the enzyme activity of the receptor kinase can be detected and, based on the detection result, it becomes possible to measure the activity of the receptor kinase.

A kinase to be measured is not particularly limited, as far as it is a receptor kinase. Specifically, examples of the receptor kinase include receptor tyrosine kinase, receptor serine/threonine kinase and the like. Examples of the receptor tyrosine kinase include growth factor receptors such as insulin receptor (IR), insulin-like growth factor receptor (IGFR), platelet-derived growth factor receptor (PDGF), fibroblast growth factor (FGFR), human epithelial growth factor receptor (HER), and vascular endothelial growth factor (VEGFR). The HER family includes HER1, HER2, HER3 and HER4.

The receptor kinase to be measured is obtained from a cell. The cell may be a cell contained in a biological sample collected from a biological body, or a cultured cell obtained from a cell line which is established from a cell collected from a biological body. A method of obtaining the receptor kinase is not particularly limited as far as it is a method of preparing a sample containing a receptor kinase by separating a cytoplasm from a cell. For example, the receptor kinase can be obtained using the following method. First, a cell membrane of a cell is fragmentated in a suitable buffer solution (hereinafter, referred to as homogenization reagent). After the solution obtained by fragmentation is separated into a supernatant and a precipitate by centrifugation, the supernatant is removed. This supernatant contains proteins derived from a cytoplasm. In the precipitate, a fragment of a cell membrane retaining the receptor kinase is contained. The precipitate and a solution containing a surfactant (hereinafter, referred to as solubilizing reagent) are mixed, and the resulting mixture is separated into a supernatant and a precipitate by centrifugation. The supernatant contains a cell membrane having a receptor kinase. The cell membrane contained in the supernatant is converted into a micelle with a surfactant. In the precipitate, insoluble proteins and a DNA are contained. And, this supernatant can be used as a sample containing a receptor kinase. In the thus prepared sample, a fragmentated cell membrane may be converted into a micelle with a surfactant, and a receptor kinase may be penetrated into the cell membrane.

In addition, it is known that, among the receptor kinases, there are kinases which form a homodimer or a heterodimer by binding with a ligand. In a sample prepared by the aforementioned method, the receptor kinase is contained in the state where a steric structure is retained to such an extent that the dimer can be formed. Therefore, by using a sample prepared by the aforementioned method, the activity of a receptor kinase can be measured more precisely. For example, in the case of HER1, two molecules of HER1 are bound by binding with EGF which is a ligand, to form a dimer. In the aforementioned sample, a dimer in the state where a steric structure is retained is contained.

The homogenization reagent is used in order to prevent denaturation of the receptor kinase when a cell is fragmentated. A pH of the homogenization reagent is not particularly limited as far as it is in such a range that the receptor kinase can be obtained in the stabilized state without denaturation and inactivation. A pH of the homogenization reagent is preferably 4.0 to 9.0, more preferably 4.5 to 8.5, further preferably 5.0 to 8.0. It is preferable that the homogenization reagent contains a buffer agent. Examples of the buffer agent include a phosphate buffer, an acetate buffer, a citrate buffer, MOPS (3-morpholinopropanesulfonic acid), HEPES (2-[4-(2-hydroxyethyl)-1-piperazinyl]ethanesulfonic acid), Tris (tris(hydroxymethyl)aminomethane), and tricin (N-[tris(hydroxymethyl)methyl]glycine). The solubilizing reagent also contains the aforementioned buffer agent, and has preferably a pH which is the same extent as that of the homogenization reagent.

To the homogenization reagent and/or the solubilization reagent may be added a protease inhibitor, a dephosphorylation enzyme inhibitor, a reagent for preventing oxidation of a thiol group (hereinafter, referred to as thiol group stabilization agent) and the like.

A method of fragmentating a cell membrane is not particularly limited as far as it can fragmentate a cell membrane. Examples of the method include suction discharge with a pipette, stirring with a vortex mixer, fragmentation with a blender, pressurization with a pestle, ultrasound treatment with an ultrasound treating apparatus, and the like.

A surfactant can be used for solubilizing a fragmented cell membrane (conversion into micelle), provided that it is preferable to use a surfactant which does not degrade or degenerate a receptor kinase contained in a cell membrane. There is a possibility that a surfactant having a charge binds to a receptor kinase, and changes a steric structure thereof. For this reason, it is preferable to use, as a surfactant, a nonionic surfactant which does not substantially bind to a receptor kinase. Examples of the nonionic surfactant include nonionic surfactants having a fundamental structure of dodecyl ether, cetyl ether, stearyl ether, p-t-octyl phenyl ether or the like. Specific examples of such the nonionic surfactant include Nonidet P-40 (NP-40, registered trademark of Shell International Petroleum Company Limited), Triton-X (registered trademark of Union Carbide Chemicals and Plastics Inc.), Tween (registered trademark of ICI Americas Inc.), Brij (registered trademark of ICI Americas Inc.), Emulgel (registered trademark of Kao Corporation) and the like. A concentration of the surfactant in the solubilizing reagent is preferably 0.05 to 5%, more preferably 0.1 to 3%, further preferably 0.1 to 1%.

The protease inhibitor can be used in order to prevent degradation of a receptor kinase with a protease contained in a cell. Examples of the protease inhibitor include a metalloprotease inhibitor such as EDTA and EGTA, a serine protease inhibitor such as PMSF, a trypsin inhibitor, chymotrypsin and the like, a cysteine protease inhibitor such as iodoacetamide, E-64 and the like. These protease inhibitors may be used alone, or may be used by mixing them. Alternatively, a sold product in which a plurality of protease inhibitors have been mixed in advance, such as a protease inhibitor cocktail (Sigma), may be used.

The dephosphorylation enzyme inhibitor can be used in order to prevent reduction in the enzyme activity of a receptor kinase with dephosphorylation enzyme contained in a cell. Examples of the dephosphorylation enzyme inhibitor include a tyrosine dephosphorylation enzyme inhibitor, a serine/threonine dephosphorylation enzyme inhibitor and the like. Examples of the tyrosine dephosphorylation enzyme inhibitor include sodium orthovanadate (Na₃VO₄). Examples of the serine/threonine dephosphorylation enzyme inhibitor include sodium fluoride (NaF). These dephosphorylation inhibitors may be used alone, or may be used by mixing them.

The thiol group stabilization agent can be used in order to prevent inactivation of a receptor kinase. A thiol group contained in an enzyme is easily oxidized to form more stable disulfide. Since formation of disulfide changes a structure of an enzyme, the formation becomes a cause for inactivation of an enzyme. Oxidation of a thiol group can be prevented with a reagent containing a SH group. Examples of the thiol group stabilization agent include dithiothreitol (DTT), 2-mercaptoethanol, glutathione, cysteine, homocysteine, coenzyme A, dihydrolipoic acid and the like. For example, when the thiol group stabilization agent is DTT, a concentration of the thiol group stabilization agent in the homogenization reagent and/or the solubilizing reagent is preferably 0.05 to 2 mM, more preferably 0.07 to 1.7 mM, further preferably 0.1 to 1.5 mM. For example, when the thiol group stabilization agent is 2-mercaptoethanol, a concentration of the thiol group stabilization agent in the homogenization reagent and/or the solubilizing reagent is preferably 0.1 to 15 mM, more preferably 0.3 to 13 mM, further preferably 0.5 to 12 mM.

By contacting a receptor kinase obtained from a cell with a substrate, an enzyme reaction can be performed. Preferably, by contacting the aforementioned sample (sample containing receptor kinase), a substrate corresponding to a receptor kinase to be measured, and a phosphate group donor by mixing them, an enzyme reaction can be performed with the receptor kinase activity.

The substrate used in the enzyme reaction is not particularly limited as far as it is phosphorylated by a receptor kinase to be measured, and is appropriately selected depending on a receptor kinase to be measured. For example, when a subject to be measured is HER1, as the substrate, Grb2, myelin basic protein (MBP), histone H2B (HH2B), phospholipase C gamma and the like can be used. Alternatively, a substrate having high specificity for a particular receptor kinase may be used. For example, when a subject to be measured is HER1, as a substrate having high specificity for HER1, a commercially available substrate such as GST EGFR-substrate (Stratagene) may be used. The GST-EGFR substrate is a fused protein of GST and a substrate which has been artificially prepared so that it is phosphorylated by the enzyme activity of HER1. By using such the substrate in measurement, it becomes possible to specifically detect the activity of HER1 among a plurality of kinds of receptor kinases.

On the other hand, when a plurality of kinds of receptor kinases are to be measured, substrates of receptor kinases to be measured may be used by combining them, or a substrate which can be phosphorylated by all of those receptor kinases may be used. Examples of the substrate which can be phosphorylated by a plurality of kinds of receptor kinases include the known synthetic peptides which have been artificially prepared so that they are phosphorylated with the enzyme activities of a plurality of kinds of receptor kinases. Examples of the substrate include synthetic peptides which are used as a substrate of a tyrosine kinase in the literature of Sasaki et al., (Norio Sasaki et al., 1985, The Journal of Biological Chemistry, Vol. 260, No.17, 9793-9804), the literature of Braun et al., (Sergei Braun et al., 1984, The Journal of Biological Chemistry, Vol.259, No.4, 2051-2054), the literature of Abdel-Ghany et al. (M.Abdel-Ghany et al.,1990, Proceeding of The National Academy of Science, Vol, 87, 7061-7065) and the like. Synthetic peptides disclosed in these literatures consist of an amino acid sequence comprising glutamic acid (hereinafter, abbreviated as Glu) and tyrosine residue (hereinafter, abbreviated as Tyr) and have been artificially prepared so that Tyr can be phosphorylated with a plurality of kinds of tyrosine kinases. As the aforementioned amino acid sequence, specifically, the following amino acid sequences can be exemplified:
an amino acid sequence in which a sequence consisting of four Glus and one Tyr is repeated two or more times (hereinafter, referred to as amino acid sequence "a"),
an amino acid sequence in which a sequence consisting of one Glu and one Tyr is repeated two or more times (hereinafter, referred to as amino acid sequence "b"),
an amino acid sequence in which a sequence consisting of six Glus, one Tyr and three alanine residues (hereinafter, referred to as Ala) is repeated two or more times (hereinafter, referred to as amino acid sequence "c"),
an amino acid sequence in which a sequence consisting of one Glu, one Tyr and one Ala is repeated two or more times (hereinafter referred to as amino acid sequence "d") and
an amino acid sequence in which a sequence consisting of two Glus and one Tyr, six Alas and five lysine residues (hereinafter, abbreviated as Lys) is repeated two or more times (hereinafter, referred to as amino acid sequence "e").

In the literature of Hunter et al. (Tony Hunter, 1982, The Journal of Biological Chemistry, Vol. 257, No.9, 4843-4848), there is a report that an acidic amino acid residue is important for phosphorylation of Tyr by a tyrosine kinase. Thereby, particularly, an amino acid sequence "a" and an amino acid sequence "c" containing many Glus which are an acidic amino acid residue are preferable. By using, as a substrate, a peptide consisting of such the amino acid sequence in measurement, it becomes possible to measure a total activity value of a receptor kinase.

In the enzyme reaction, a phosphate group of a phosphate group donor is incorporated into a substrate by the enzyme activity of a receptor kinase which has been activated by autophosphorylation. For example, when a tyrosine kinase is to be measured, a tyrosine residue in a substrate is phosphorylated.

Examples of the phosphate group donor include adenosine triphosphate (ATP), adenosine 5'-O-(3-thiotriphosphate)(ATP-γS), 32P-labeled adenosine 5'-O- (3-triphosphate) (γ- [32P] -ATP), adenosine diphosphate (ADP), adenosine monophosphate (AMP) and the like.

It is preferable that a substrate used in the enzyme reaction has an affinity tag. Using the affinity tag and a solid phase having a binding substance which can bind to the affinity tag (hereinafter, referred to as solid phase), a substrate can be obtained. Specifically, by obtaining a complex in which a substrate having the affinity tag and the solid phase are bound, and dissociating a bond between the affinity tag and the binding substance in the obtained complex, a substrate can be obtained finally.

The affinity tag is not particularly limited as far as it is a substance which can bind to the binding substance and does not interfere with binding of a substrate to a receptor kinase and phosphorylation of a substrate. For example, a polypeptide, a hapten and the like can be used. As the affinity tag, specifically, glutathione-S-transferase (hereinafter, referred to as GST), histidine, maltose binding protein, FLAG peptide (Sigma), Myc tag, HA tag, Strep tag (IBA bMBH), biotin, avidin, streptoavidin and the like can be used.

As the substrate having the affinity tag, for example, a fused protein of a substrate and an affinity tag can be used. As the fused protein, an entity in which an affinity tag and a substrate are bound may be used. In addition, as the fused protein, a fused protein obtained by introducing, into a host, a vector having a recombinant gene expressing a fused protein of an affinity tag and a substrate, and making the host produce it, may be used. When HER1 is to be measured, the aforementioned GST-EGFR substrate is suitably used.

The binding substance is not particularly limited as far as it can dissociably bind to an affinity tag. Examples of the binding substance include glutathione, nickel, amylose, FLAG antibody (Sigma), Myc antibody, hemagglutinin (HA antibody, Strep-Tactin (IBA GmbH) and the like.

The solid phase is not particularly limited as far as it is a carrier which can bind to a binding substance. Examples of a material for the solid phase include polysaccharides, plastics, glasses and the like. Examples of a shape of the solid phase include beads, gels and the like. Examples of the solid phase include Sepharose beads, agarose beads, magnetic beads, glass beads, silicone gels and the like. And, the beads and gels may be used by filling into a column.

When the substrate is the GST-EGFR substrate as described above, as the solid phase, for example, glutathione Sepharose beads (hereinafter, referred to as glutathione beads) can be used. In that case, the GST-EGFR substrate phosphorylated by the enzyme activity of a receptor kinase and glutathione beads are bound. Glutathione beads bound to the GST-EGFR substrate are collected, and reducing glutathione is added to the collected glutathione beads, and thereby, a bond between GST and glutathione beads can be dissociated. Thereby, a phosphorylated GST-EGFR substrate can be obtained.

When a phosphorylated GST-EGFR substrate is obtained, a GST-EGFR substrate and glutathione beads are bound in advance, and this may be used in the enzyme reaction, or a GST-EGFR substrate and glutathione beads may be bound after the enzyme reaction.

Examples of a combination of the affinity tag and the solid phase in addition to the aforementioned combinations include the following examples.

When histidine is selected as the affinity tag, as the solid phase, for example, nickel agarose beads can be used. A bond between histidine and nickel can be dissociated using, for example, an acid such as Glycine-HCl and the like, or imidazole.

When a maltose-binding protein is selected as the affinity tag, as the solid phase, for example, amylose magnetic beads can be used. A bond between the maltose-binding protein and amylose can be dissociated, for example, by adding free amylose.

When the FLAG peptide is selected as the affinity tag, as the solid phase, the FLAG affinity gel of Sigma can be used. A bond between the FLAG peptide and the FLAG affinity gel can be dissociated by, for example, using an acid such as Glycine-HCl and the like, or the 3xFLAG peptide (Sigma).

When the Myc tag is selected as the affinity tag, as the solid phase, for example, agarose beads bound to the Myc antibody can be used. In addition, when the HA tag is selected as the affinity tag, agarose beads bound to the HA antibody can be used. Both of a bond between the Myc tag and the Myc antibody, and a bond between the HA tag and the HA antibody can be dissociated, for example, by denaturing a protein by adding an acid or an alkali. Thereupon, it is preferable to select an acid or an alkali which can return the denatured protein to the original state. Specifically, examples of the acid include hydrochloric acid and the like, and examples of the alkali include sodium hydroxide and the like.

When the Strep tag is selected as the affinity tag, as the solid phase, the Strep-Tactin solid phased gel column of GmbH can be used. A bond between the Strep tag and Strep-Tactin can be dissociated, for example, using desthiobiotin which reversibly reacts streptoavidin.

After a receptor tyrosine kinase in a sample and a substrate are enzyme-reacted, and before the substrate is obtained, the enzyme reaction may be stopped using heat treatment, cooling treatment, or a kinase inhibitor. In a step of obtaining a substrate, an enzyme reaction further proceeds in some cases, and this may be a cause for generation of scatter in measurement result for every sample. However, by stopping the enzyme reaction before obtaining of the substrate, this can be avoided. Examples of the HER1 inhibitor include PD168393, PD153035 (all Carbiochem) and the like.

For detecting a phosphorylation substrate, a labeling substance is used. Examples of the labeling substance are not limited to, but include a fluorescent substance, an enzyme, a radioactive isotope and the like. Examples of the fluorescent substance include fluorescein, coumarin, eosin, phenanthroline, pyrene, rhodimine and the like. Examples of the enzyme include alkaline phosphatase, peroxidase and the like. Examples of the radioactive isotope include ³²P, ³³P, ¹³¹I, ¹²⁵I, ³H, ¹⁴C, ³⁵S and the like.

For detecting a phosphorylation substrate, a labeling substance is bound to the phosphorylation substrate. For example, by using an antibody which has a labeling substance and can specifically bind to the phosphorylation substrate, the labeling substance can be bound to the phosphorylation substrate.

Alternatively, by using an antibody which can specifically bind to a phosphorylation substrate (hereinafter, referred to as phosphorylating substrate-specific antibody) and an antibody which can bind to the phosphorylating substrate-specific antibody and has a labeling substance (hereinafter, referred to as secondary antibody), the labeling substance can be bound to the phosphorylation substrate. In this case, the labeling substance can substantially bind to the phosphorylation substance via the phosphorylating substrate-specific antibody and secondary antibody.

Alternatively, by using a phosphorylating substrate-specific antibody, a secondary antibody having biotin and avidin having a labeling substance, the labeling substance can bind to the phosphorylation substrate. In this case, the labeling substance can substantially bind to the phosphorylation substrate via the phosphorylating substrate-specific antibody, the secondary antibody, biotin and avidin. Alternatively, the secondary antibody may have avidin, and biotin may have the labeling substance.

Alternatively, a phosphorylating substrate-specific antibody having biotin and avidin having a labeling substance may be used. Alternatively, a phosphorylating substrate-specific antibody having avidin and biotin having a labeling substance may be used.

By detecting the labeling substance, a phosphorylated substrate can be detected, and thereby, the activity of a receptor kinase can be finally measured.

As the aforementioned phosphorylating substrate-specific antibody and secondary antibody, an antibody obtained by promoting immunization by contacting an animal with an antigen, and purifying blood of this animal, an antibody obtained by gene recombination, a polyclonal antibody, a monoclonal antibody and the like can be used. Alternatively, among these antibodies, a mixture of at least two kinds may be used. As used herein, the antibody includes a fragment of an antibody, and a derivative thereof. Specific examples of the antibody include a Fab fragment, a F(ab') fragment, a F(ab)₂ fragment, and a sFb fragment (Blazar et al., 1997, Journal of Immunology, 159:5821-5833 and Bird et al., 1988, Science, 242:423-426). Examples of a class of the antibody to be used are not limited to, but include IgG and IgM.

A method of detecting the phosphorylation substrate is appropriately selected depending on a kind of the labeling substance. When the labeling substance is a fluorescent substance, phosphorylation of a substrate can be detected by Western blotting. The phosphorylation substrate is separated by a membrane, a phosphorylating substrate-specific antibody is added to bind to the phosphorylation substrate, a secondary antibody having a fluorescent substance is further bound to the phosphorylating substrate-specific antibody, and fluorescence thereof may be detected. When the phosphorylation substrate is separated in advance using the aforementioned affinity tag, phosphorylation of the substrate may be detected using a Slot blot method in place of Western blotting. As the labeling substance, an enzyme may be used in place of the fluorescent substance. In this case, an enzyme possessed by a secondary antibody is subjected to a color development reaction by adding a substrate, and color development thereof may be detected.

Alternatively, by accommodating a solution containing a phosphorylation substrate in a tube, adding a phosphorylating substrate-specific antibody having a fluorescent substance to bind to the phosphorylation substrate, and measuring a fluorescent intensity, phosphorylation of a substrate may be detected.

When the labeling substance is an enzyme, phosphorylation of a substrate may be detected by a solid phase enzyme immunoassay (hereinafter, referred to as ELISA method) . The ELISA method includes a direct adsorption method and a sandwich method.

In the direct adsorption method, a phosphorylation substrate is adsorbed onto a surface of a solid phase, and a phosphorylating substrate-specific antibody having an enzyme is added to bind to the phosphorylation substrate. Then, the enzyme possessed by the phosphorylating substrate-specific antibody is subjected to a color developing reaction by addition of a substrate, and color development thereof may be detected.

In the sandwich method, a phosphorylating substrate-specific antibody is bound to a solid phase (hereinafter, referred to as solid-phased antibody), and a phosphorylation substrate is added to bind to the solid phase antibody. Then, a phosphorylating substrate-specific antibody having an enzyme (hereinafter, referred to as labeling antibody) is added to bind to the phosphorylation substrate. The enzyme possessed by the labeling antibody is subjected to a color development reaction by addition of a substrate, and color development thereof may be detected.

For example, when the enzyme is alkaline phosphatase, as a substrate, a mixed solution of nitrotetrazolium blue chloride (NBT) and 5-bromo-4-chloro-3-indoxyl phosphite (BCIP) may be used to perform a reaction, to develop a color. When the enzyme is peroxidase, as a substrate, diaminobenzidine (DAB) may be used to perform a reaction, and to develop a color.

When the sandwich method is used, it is preferable that the solid-phased antibody and the labeling antibody are bound to different sites of a phosphorylation substrate. That is, it is preferable that there are a plurality of antibody binding sites on the phosphorylation substrate or two kinds of antibodies used recognize different antigen determinants of the phosphorylation substrate.

When the labeling substance is a radioactive isotope, phosphorylation of a substrate can be detected by radioimmunoassay (hereinafter, referred to as RIA). Specifically, a phosphorylating substrate-specific antibody having a radioactive isotope is bound to a phosphorylation substrate, radiation is measured with a scintillation counter or the like, and thereby, phosphorylation of a substrate can be detected.

Based on the measurement result obtained by using the method of the present embodiment, the presence or the absence of malignant alteration of a cell may be presumed. Previously, a method of measuring an expression amount of a protein involved in malignant alteration of a cell and determining the presence or the absence of a malignant cell has been known. However, these proteins cause malignant alteration in many cases when the activity is high or low, and it is difficult to determine malignant alteration only by a more or less expression amount. Therefore, the result of measurement of the kinase activity obtained by using the method of the present embodiment may be information by which the presence or the absence of malignant alteration of a cell can be presumed, unlike the case where an expression amount of a kinase is merely measured. Alternatively, both of an activity value and an expression amount of a kinase may be measured.

Based on the result of measurement obtained by using the method of the present embodiment, a cause for malignant alteration of a cell may be presumed. For example, using the method of the present embodiment, a total activity value of a receptor tyrosine kinase present in a cell membrane is measured, and this total activity value may be information by which whether a receptor tyrosine kinase is a cause for malignant alteration or not can be presumed. Specifically, using a substrate which can be phosphorylated by a plurality of kinds of receptor tyrosine kinases in the method of the present embodiment, a total activity value of a receptor tyrosine kinase present in a cell membrane of a malignant cell is measured, the total activity value and a threshold corresponding thereto are compared and, when the total activity value is not less than the threshold, the receptor tyrosine kinase may be presumed to be a cause for malignant alteration and, when the value is less than the threshold, the receptor tyrosine kinase may be presumed not to be a cause for malignant alteration. For example, the threshold can be obtained using the following method. First, a total activity value of a receptor tyrosine kinase of a tumor cell collected from each of a cancer patient population "A" in which a receptor tyrosine kinase is a cause for malignant alteration, and a cancer patient population "B" in which a receptor tyrosine kinase is not a cause for malignant alteration is measured. The total activity values of respective cancer patient populations are compared. A total activity value by which a cancer patient population "A" and a cancer patient population "B" can be classified at a high efficiency can be adopted as a threshold.

Based on the result obtained by using a method of presuming influence of an inhibitor which inhibits the activity of the receptor kinase of the present embodiment (hereinafter, referred to as kinase inhibitor), information by which the inhibiting ability of a kinase inhibitor can be presumed, may be obtained. Examples of the kinase inhibitor include an anti-cancer agent exhibiting a drug efficacy by inhibiting the kinase activity. Examples of such the anti-cancer agent include Iressa (AstraZeneca) and Herceptin (Genentech). Iressa is an inhibitor of HER1, and Herceptin is an inhibitor of HER2.

Examples of a method of presuming the inhibiting ability of the kinase inhibitor include the following method. First, a kinase activity value of a cell is measured. Then, this cell is contacted with an inhibitor, and a kinase activity value of the cell after the contact is measured. A way to obtain an activity value is different depending on a labeling substance and a detection method. For example, when a fluorescent substance is used as the labeling substance, an activity value can be obtained by measuring a fluorescent intensity. A kinase activity value of a cell before contact with a kinase inhibitor and a kinase activity value of a cell after contact with the kinase inhibitor are compared. When significant reduction in an activity value is recognized, it may be presumed that the kinase activity was inhibited by contact with the inhibitor. When significant reduction in the activity value is not recognized, it may be presumed that there is no influence of contact with the inhibitor.

Based on the result obtained by using a method of presuming influence of a ligand which activates a receptor kinase of the present embodiment, information by which the presence of the receptor kinase corresponding to a ligand can be presumed, may be obtained. As the ligand, the known ligand for the receptor kinase can be used. Examples of the ligand include epithelial growth factor (EGF), Heregulin, insulin-like growth factor (IGF), fibroblast growth factor (FGF), vascular endothelial growth factor (VEGF), and platelet-derived growth factor (PDGF). EGF and Heregulin bind to HER, IGF binds to IGFR, FGF binds to FGFR, VEGF binds to VEGFR, and PDGF binds to PDGFR. When the ligand binds to a receptor kinase, dimerization of the receptor kinase is induced. When a dimer of the receptor kinase is formed, a tyrosine kinase site present in an intracellular domain of the receptor kinase is activated.

Examples of a method of presuming of the presence of a receptor kinase corresponding to the ligand include the following method. First, a kinase activity value of a cell is measured. Then, this cell and a ligand are contacted, and a kinase activity value of the cell after the contact is measured. A way to obtain an activity value is different depending on a labeling substance and a detection method. For example, when a fluorescent substance is used as the labeling substance, an activity value can be obtained by measuring a fluorescent intensity. A kinase activity value of a cell before contact with the ligand and a kinase activity value of a cell after contact with the ligand are compared. When significant increase in an activity value is recognized, it may be presumed that a receptor kinase corresponding to the ligand is present in a cell and this is activated by contact with the ligand.

Reagents used in measuring the activity of the receptor kinase can be formulated into a reagent kit. This kit comprises: a substrate corresponding to a receptor kinase, a phosphate group donor comprising a phosphate group which can be introduced into the substrate by the activity of the receptor kinase, and a labeling substance which can bind to the substrate in which a phosphate group has been introduced. The above components may be accommodated in a single container, or at least one component may be accommodated in another container. Preferably, a reagent containing the substrate and the phosphate donor is accommodated in a first container, and a reagent containing a labeling substance is accommodated in a second container. In addition, when the labeling substance consists of a primary antibody which can bind to a substrate and a secondary antibody which can bind to the primary antibody and has a labeling substance, it is preferable that the primary antibody and secondary antibody are accommodated in separate containers, respectively. For adjusting a pH, a buffer agent may be contained in the reagent. As the buffer agent, the aforementioned buffers can be used.

The reagent kit may comprise a homogenization reagent and/or a solubilizing reagent.

The method of measuring the activity of a receptor kinase of the present invention will be explained more specifically below based on Examples. The present invention is not limited to these Examples.

### (Example 1) Detection of phosphorylation of substrate by activation of HER1

### 1. Method of preparing sample for reaction

A culturing cell (MDA-MB468) derived from a breast cancer was cultured to 80% confluent (about 107 cells) in a 225 cm² flask. This cultured cell and 1ml of a cell treating solution (containing 20 mM HEPES pH 7.4, 20 mM MnCl₂, 1 mM DTT, 0.2% protease inhibitor (hereinafter, referred to as PI), 10% glycerol, 100 µM Na₃VO₄, and 50 mM NaF) were mixed. Then, the obtained mixture was pressurized by using a pestle to destruct a cell membrane in the mixed solution, to prepare a cell solution. As PI, a protease inhibitor cocktail For Mammalian Tissues (Sigma) was used. The resulting cell solution was centrifuged, and the supernatant was discarded. A precipitate and a cell membrane solubilizing solution (containing 20 mM HEPES pH 7.4, 20 mM MnCl₂, 1 mM DTT, 1% NP-40, 0.2% PI, 10% glycerol, 100 µM Na₃VO₄, and 50 mM NaF) were mixed. By pressurizing the obtained mixture by using a pestle, a cell membrane was solubilized, and centrifuged, and the supernatant was recovered. This supernatant was used as a sample for a reaction in the following reaction.

### 2. Enzyme reaction and elution of reaction substrate

### i) Preparation of SAMPLE-1 and SAMPLE-2

25 µl of the sample for a reaction obtained by the above method, and 25 µl of a substrate solution 1 (containing 20 mM HEPES pH 7.4, 10 mM MnCl₂, 1 mM DTT, 0.1% NP-40, 0.2% PI, 10% glycerol, 100 µM Na₃VO₄, 50 mM NaF, 200 µM ATP, and 5 µg of substrate) were mixed, followed by incubation at 25°C for 30 minutes. This was designated as SAMPLE-1.

25 µl of the sample for a reaction, and 25 µl of a substrate solution 2 (containing 20 mM HEPES pH 7.4, 10 mM MnCl₂, 1 mM DTT, 0.1% NP-40, 0.2% PI, 10% glycerol, 100 µM Na₃VO₄, and 5 µg of substrate) were mixed, followed by incubation at 25°C for 30 minutes. This was designated as SAMPLE-2. As a substrate, a GSTEGFR-substrate (Stratagene) bound to Sepharose 4B beads (Amersham) having a particle diameter of 45 to 165 µm was used.

### ii) Preparation of SAMPLE-3 and SAMPLE-4

SAMPLE-1 or SAMPLE-2, and 50 µl of a reaction stopping solution (containing 20 mM HEPES pH 7.4, 10 mM MnCl₂, 1 mM DTT, 0.1% NP-40, 0.2% PI, 10% glycerol, 100 µM Na₃VO₄, and 10 µM PD168393 (EGFR inhibitor, Carbiochem)) were mixed, respectively, followed by centrifugation. The supernatant of the mixture obtained from SAMPLE-1 was designated as SAMPLE-3, and the supernatant of the mixture obtained from SAMPLE-2 was designated as SAMPLE-4.

### iii) Preparation of SAMPLE-5 and SAMPLE-6

After the supernatant was discarded, a precipitate of SAMPLE-3 and a precipitate of SAMPLE-4, and 200 µl of a first washing solution (containing 20 mM HEPES pH 7.4, 10 mM MnCl₂, 1 mM DTT, 0.1% NP-40, 0.2% PI, 10% glycerol, 100 µM Na₃VO₄, and 100nM PD168393) were mixed, respectively, followed by centrifugation. After the supernatant was discarded, a precipitate of SAMPLE-3 or a precipitate of SAMPLE-4, and 200 µl of a second washing solution (containing 25 mM Tris pH 7.4, 0.2% PI, 100 µM Na₃VO₄, and 150 mM NaCl) were mixed, respectively, followed by centrifugation. After the supernatant was discarded, a precipitate of a SAMPLE-3 or a precipitate of a SAMPLE-4, and 190 µl of an eluent (containing 50 mM Tris pH 8.0, 10 mM reducing glutathione, 100 µM Na₃VO₄, and 0.2% PI) were mixed, respectively. The mixture of a precipitate of SAMPLE-3 and an eluent was designated as SAMPLE-5, and the mixture of a precipitate of SAMPLE-4 and an eluent was designated as SAMPLE-6.

### iv) Preparation of SAMPLE-7 and SAMPLE-8

SAMPLE-5 and SAMPLE-6 were centrifuged. The supernatant of SAMPLE-5 was designated as SAMPLE-7, and the supernatant of SAMPLE-6 was designated as SAMPLE-8.

### v) Preparation of SAMPLE-9 and SAMPLE-10

A precipitate of SAMPLE-5 obtained in iv was designated as SAMPLE-9, and a precipitate of SAMPLE-6 was designated as SAMPLE-10.

### 3. Detection of phosphorylated substrate by Western blotting Proteins contained in SAMPLE-1 to-10 were separated by SDS-PAGE. Each 12.5 µl of a SDS sample buffer pH 6.8 (containing 200 mM Tris, 40% glycerol, 8% SDS, and 10% 2-mercaptoethanol) was added to SAMPLE-1 and SAMPLE-2, each 10 µl of the sample buffer was added to SAMPLE -3 to 6, 9 and 10, and each 5 µl of the sample buffer was added to SAMPLE -7 and 8, followed by boiling at 100°C for 5 minutes. Each SAMPLE was injected into each well of a polyacrylamide gel (PAG MINI "First" 4/20 (13W) (Daiich Pure Chemicals Co. , Ltd.)), and this was electrophoresed at 25mA for 70 minutes using a electrophoresis tank (cassette electrophoresis tank "First" DPE-1020 (mini duplicate) (Daiich Pure Chemicals Co., Ltd.)). A voltage was applied to proteins separated by electrophoresis, at 100V for 1 hour using a mini trans blot cell (Biorad), to transfer them from a polyacrylamide gel to a polyvinylidene fluoride (PVDF) membrane (Immobilon-FL 0.45 µm pore size (Millipore)).

This PVDF membrane was blocked with a 4% Block Ace (Dainippon Sumitomo Pharma Co., Ltd.) solution for 60 minutes. The blocked PVDF membrane was shaken for 60 minutes in 2ml of a primary antibody solution (containing 0.4% Block Ace and 0.5 µg/ml Anti-Phosphotyrosine clone 4G10 (Upstate)), and washed with TBS-T (containing 25 mM Tris, 150 mM NaCl and 0.1% Tween-20) three times. Then, this PVDF membrane was shaken for 60 minutes in 2ml of a secondary antibody solution (containing 0.4% Block Ace and 2.7 µg/ml anti-mouse immunoglobulin • rabbit polyclonal antibody FITC label (DAKO)), and washed with TBS-T three times. This PVDF membrane was dried, and analyzed using an image analyzing apparatus (Pharos FX system (Biorad)), and fluorescence was detected.

### 4. Results

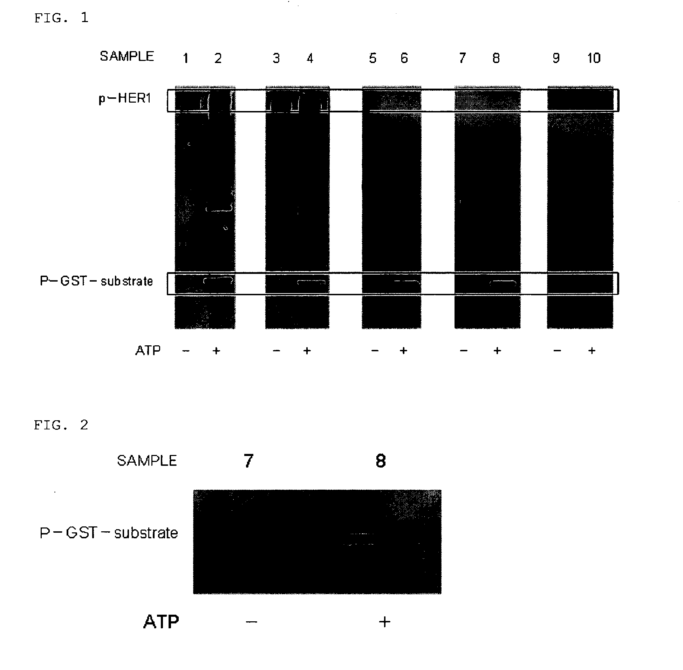

Fig. 1 is a fluorescent photograph showing results of Western blotting. In each photograph of SAMPLE-1 to -10, as SAMPLE having an odd number, a reaction solution containing no ATP was used. As SAMPLE having an even number, a reaction solution containing ATP was used.

No band was seen in SAMPLE-1, 3, 5 and 7 because it is thought that ATP is not contained in the reaction solution, and a substrate was not phosphorylated. It is thought that phosphorylation in SAMPLE-1,and SAMPLE-3 is due to donation of a phosphate group from endogenous ATP.

A band seen in a frame of p-HER1 is autophosphorylated HER1. A band seen in a frame of p-GST-substrate is a substrate (EGFR-substrate) phosphorylated by activation of HER1. A band seen at an approximate center of lanes of SAMPLE-2 and SAMPLE-4 is thought to be any protein having phosphorylated tyrosine.

From results of SAMPLE-2 and 4, it was seen that even when a plurality of phosphorylation substrates are present in admixture thereof, they can be separated by Western blotting, and phosphorylation of an objective substrate can be detected. In addition, it was seen that a phosphorylation substrate is contained in the supernatant of a reaction solution obtained by centrifugation after an enzyme reaction.

SAMPLE-5 and 6 are such that proteins other than a substrate bound to Sepharose beads are removed, and a substrate bound to Sepharose beads is recovered. From results of SAMPLE-6, it was seen that only a phosphorylation substrate bound to Sepharose beads can be recovered.

SAMPLE-7 and 8 are such that a substrate bound to Sepharose beads is eluted, and a substrate is recovered. From results of SAMPLE-8, it was seen that a phosphorylation substrate is dissociated from beads, and only a phosphorylation substrate can be recovered.

SAMPLE-9 and SAMPLE-10 are Sepharose beads after elution of proteins, and a band showing a protein containing phosphorylated tyrosine was not seen. From this result, it was seen that a bond between GST possessed by a substrate and Sepahrose beads can be dissociated.

From the forgoing, it was seen that the presence or the absence of phosphorylation of a substrate by activation of a kinase can be detected using the measuring method of the present Example.

### (Example 2) Detection of phosphorylated substrate by Slot blot

Using SAMPLE-7 and SAMPLE-8 prepared in Example 1, phosphorylated tyrosine was detected with Slot blot.

Each 180 µl of SAMPLE-7 and SAMPLE-8 were accommodated in separated wells of a slot blotter (BIO-DOT SF (Biorad)) having a PVDF membrane (Immobilon-P SQ 0.2 µm pore size (Millipore)) on a bottom. SAMPLE-7 and SAMPLE-8 were sucked from a back side of the PVDF membrane using a vacuum pump to adsorb proteins contained in each SAMPLE onto a PVDF membrane. The PVDF membrane was removed from the slot blotter, shaken for 60 minutes in 2ml of the same primary antibody solution as that of Example 1, and washed with TBS (containing 25 mM Tris and 150 mM NaCl) three times. Then, this PVDF membrane was shaken for 60 minutes in 2ml of the same secondary antibody solution as that of Example 1, and washed with TBS three times. The PVDF membrane was dried, and analyzed using an image analyzing apparatus, and fluorescence was detected.

Fig. 2 is a fluorescent photograph showing results of Slot blot. Left is blotting results of SAMPLE-7, and right is blotting results of SAMPLE-8. In SAMPLE-7, a signal showing phosphorylation could not be confirmed because it is thought that ATP was not contained in the reaction solution, a phosphate group to be incorporated into a substrate by the kinase activity of HER1 was deficient, and phosphorylation of a substrate did not occur. In SAMPLE-8, a signal showing phosphorylation could be confirmed because it is thought that a phosphate group of ATP contained in the reaction solution was incorporated into a substrate by the kinase activity of HER1, and phosphorylation occurred.

From the forgoing results, it was seen that the presence or the absence of phosphorylation of a substrate by activation of a kinase can be detected by Slot blot.

### (Example 3) Influence of HER1 inhibitor on kinase activity of HER1

Using the sample for a reaction obtained by the same method as that of Example 1, influence of a HER1 inhibitor on the kinase activity of HER1 was investigated.

25 µ of the sample for a reaction was accommodated in two tubes. To one of tubes was added 25 µl of a reaction solution (containing 20 mM PIPES pH 7.4, 10 mM MnCl₂, 1 mM DTT, 0.1% NP-40, 0.2% PI, 10% glycerol, 100 µM Na₃VO₄, 20 µM ATP, and 3 µg of Grb2) containing 200nM PD168393 (Carbiochem) which is a HER1 inhibitor, and to the other of tubes was added 25 µl of a reaction solution containing no PD168393, followed by incubation at 25°C for 30 minutes. A reaction solution containing PD168393 was designated as PD+, and a reaction solution containing no PD168393 was designated as PD-. Using PD+ and PD-, Western blotting was performed according to the same manner as that of Example 1.

Grb2 was prepared by the following method. PCR was performed by using a human cDNA, Proof Start (Qiagen), a forward primer (5' CGCGGATCCCATATGGAAGCCATCGCCAAATATG 3': SEQ ID NO: 1) and an outer primer (5' CCCAAGCTTTTAGACGTTCCGGTTCAC 3' : SEQ ID NO: 2) which were designed based on a nucleotide sequence of Grb2 mRNA. The resulting amplification product (hereinafter, referred to as Grb2 cDNA) and a plasmid vector, pGEM3 Z (Promega) were treated with a restriction enzyme (Sma) . The Grb2 cDNA was incorporated into pGEM3Z using a ligation kit (TAKARA), to prepare a recombinant plasmid for amplification. This recombinant plasmid for amplification was transformed into Escherichia coli (DH5-α) for plasmid amplification, and this Escherichia coli was cultured for 16 hours. The cultured Escherichia coli was lysed to recover the recombinant plasmid for amplification.

The recovered recombinant plasmid for amplification, and a plasmid vector, pET-28a (+) (Novagen) was treated with a restriction enzyme (EcoRI, HindIII). From the treated recombinant plasmid for amplification, the incorporated Grb2 cDNA was recovered. Grb2cDNA was incorporated into pET-28A-c (+) using a ligation kit (TAKARA) to prepare a recombinant plasmid for expression. The recombinant plasmid for expression was transformed into Escherichia coli (BL21-AI) for expression, and this Escherichia coli was cultured for 16 hours. To the cultured Escherichia coli were added 1 mM IPTG and 0.2% arabinose (both, concentration in culturing solution), followed by further culturing for 4 hours to induce expression. Then, Escherichia coli was lysed, and Grb2 was recovered using nickel agarose beads. Since a concentration of the resulting Grb2 was 0.6 µg/µl, 5 µl was used in a reaction solution.

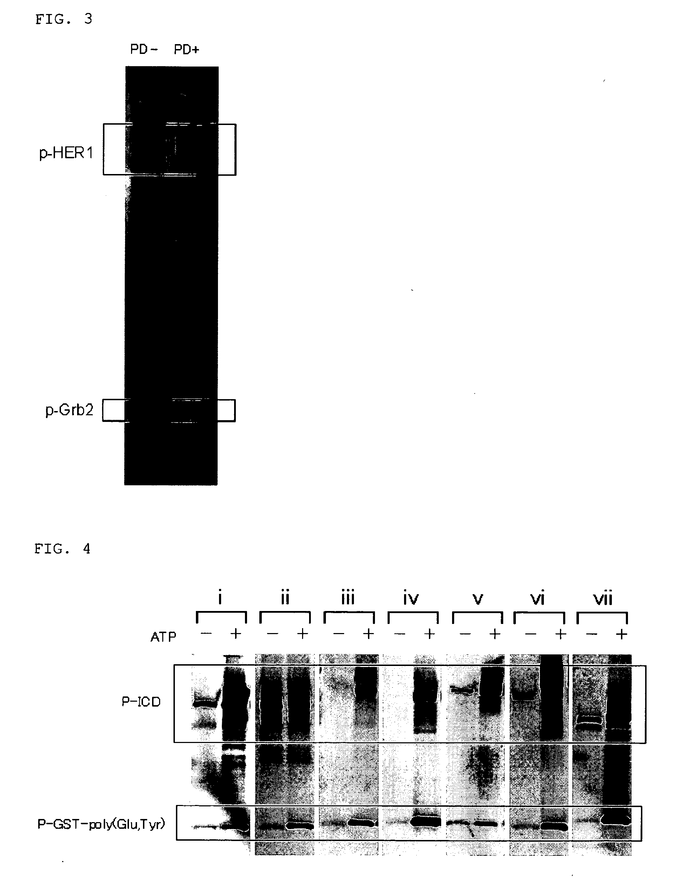

Fig. 3 is a fluorescent photograph showing results of Western blotting. A left lane is blotting result of PD-, and a light lane is blotting result of PD+.

A band seen in a frame of p-HER1 is autophosphorylated HER1. Also, in a lane of PD+, a band showing phosphorylation appears, but it is thought that phosphorylation which had already occurred before preparation of a cell as a reaction sample was detected as a signal. A band seen in a frame of p-Grb2 in a lane of PD- is Grb2 phosphorylated by the enzyme activity of HER1. A band is not seen in a frame of p-Grb2 of a lane of PD+ because it is thought that the enzyme activity of HER1 was inhibited by PD168393.

It is thought that a band seen at an approximate center of a lane of PD- is any protein phosphorylated with activation of HER1. On the other hand, a band is not seen at the same position of a lane of PD+ because it is thought that the enzyme activity of HER1 was inhibited by PD168393. From the forgoing result, using a measuring method of the present Example, it was seen that the inhibiting ability of a HER1 inhibitor on the enzyme activity of HER1 can be presumed.

Then, a substrate for a plurality of kinds of receptor tyrosine kinases was prepared, and Example of a measuring method of the present embodiment using the substrates will be shown below.

### (Example 4) Preparation of substrate for tyrosine kinase

A fused protein of GST and a peptide consisting of an amino acid sequence (SEQ ID No: 3) in which a sequence consisting of four glutamic acid residues and one tyrosine residue is repeated five times (hereinafter, referred to as poly (Glu,Tyr) peptide)was prepared. This fused protein was used as a substrate which can be phosphorylated by a plurality of kinds of tyrosine kinases. Hereinafter, this fused protein is referred to as GST-poly (Glu,Tyr) fused protein.

The GST-poly (Glu, Tyr) fused protein was prepared by the following method. PCR was performed by using KODplusDNApolymerase (Toyobo Co., Ltd.), a DNA (SEQ ID NO:4) encoding an amino acid sequence (SEQ ID NO:3) of the poly (Glu, Tyr) peptide, a sense primer (SEQ IDNO:5) and an antisense primer (SEQ ID NO:6) designed based on a nucleotide sequence of the DNA. The amplification product (hereinafter, referred to as poly (Glu,Tyr)DNA) obtained by PCR, and pGEX-4T-3 (GE Health Care Bioscience) which is a plasmid vector for GST fused protein expression were treated with a restriction enzyme (BamH1 and EcoR1) and the poly (Glu,Tyr) DNA was incorporated into pGEX-4T-3 to prepare a recombinant plasmid. This recombinant plasmid was transformed into Escherichia coli GM109, and this Escherichia coli was cultured in a liquid medium (LB medium) until an absorbance (600nm) of a culturing solution became 0.6. To this cultured Escherichia coli was added 1 mM IPTG (concentration in culturing solution), and this was cultured for 4 hours to induce expression. Then, Escherichia coli was lysed, and GST-poly (Glu,Tyr) fused protein was recovered using glutathione Sepharose 4B (GE Health Care Bioscience). An amino acid sequence of this GST-poly (Glu,Tyr) fused protein is shown in SEQ ID NO:7.

### (Example 5) Detection of phosphorylation of fused protein by Western blotting when intracellular domain of receptor tyrosine kinase is used

Herein, the GST-poly (Glu,Tyr) fused protein prepared in Example 4 was phosphorylated by using an intracellular domain (ICD) of a commercially available receptor tyrosine kinase. The phosphorylated GST-poly (Glu,Tyr) fused protein was detected by Western blotting. The receptor-type tyrosine kinase is composed of an extracellular domain, a transmembrane domain, and an intracellular domain, and a site showing the activity of tyrosine kinase is present in the intracellular domain.

### 1. Method of preparing sample for reaction

50 µl of a buffer 1 (containing 20 mM HEPES pH7.4, 10 mM MnCl₂, 1% NP40, 1 mM DTT, 0.2% protease inhibitor (hereinafter, referred to as PI), 10% glycerol, 200 µM Na₃VO₄ and 50 mM NaF) and 0.5pmol of ICD of a commercially available receptor-type tyrosine kinase were mixed, and the mixture was used as a sample for a reaction in the following enzyme reaction. In the present Example, as ICD, PDGF Receptor β Kinase (hereinafter, referred to as PDGFR-β), VEGF Receptor 1 Kinase (hereinafter, referred to as VEGFR1), VEGF Receptor 2 Kinase (VEGFR2), EGF Receptor 1 Kinase (hereinafter, referred to as HER1), ErbB2 Kinase (hereinafter, referred to as HER2), ErbB4 Kinase (hereinafter, referred to as HER4) , and IGF-1 Receptor Kinase (IGF1R) (all, Cell Signaling Technology) were used. A mixture of a buffer 1 and PDGFR-β is designated as a sample for reaction "i", a mixture of a buffer 1 and VEGFR1 is designated as a sample for reaction "ii", a mixture of a buffer 1 and VEGFR2 is designated as a sample for reaction "iii", a mixture of a buffer 1 and HER1 is designated as a sample for reaction "iv", a mixture of a buffer 1 and HER2 is designated as a sample for reaction "v", a mixture of a buffer 1 and HER3 is designated as a sample for reaction "vi", and a mixture of a buffer 1 and IGF1R is designated as a sample for reaction "vii".

### 2. Enzyme reaction

25 µl of the sample for a reaction "i" and 25 µl of a substrate solution 3 (containing 20 mM HEPES pH 7. 4, 10 mM MnCl₂, 1 mM DTT, 1% NP40, 0.2% PI, 10% glycerol, 200 µM Na₃VO₄, 50 mM NaF, 40 µM ATP, and 5 µg of GST-poly (Glu, Tyr) fused protein) were mixed, followed by incubation at 25°C for 60 minutes. To this reaction solution was added 25 µl of the SDS sample buffer used in Example 1, and the mixture was boiled at 100°C for 5 minutes to stop the enzyme reaction. The thus prepared solution is designated as a sample for SDS "i (+) ". Similarly, samples for SDS "ii(+)" to "vii(+)" were prepared from the samples for reaction "ii" to "vii".

Separately, 25 µl of the sample for a reaction "i", and 25 µl of a substrate solution 4 (containing 20 mM HEPES pH 7.4, 10 mM MnCl₂, 1 mM DTT, 1% NP40, 0.2% PI, 10% glycerol, 200 µM Na₃VO₄, 50 mM NaF, 5 µg of GST-poly(Glu,Tyr) fused protein) were mixed, followed by incubation at 25°C for 60 minutes. To this reaction solution was added 25 µl of the SDS sample buffer used in Example 1, and the mixture was boiled at 100°C for 5 minutes to stop the enzyme reaction. The thus prepared solution is designated as a sample for SDS "i (-) ". Similarly, samples for SDS "ii(-)" to "vii(-)" were prepared from the samples for reaction "ii" to "vii". The substrate solution 4 has the same composition as that of the substrate solution 3 except that ATP is not contained. And, the samples for SDS "i(-)" to "vii(-)" were used as a negative control of the samples for SDS "i(+)" to "vii(+)".

### 3. Detection of fused protein phosphorylated with Western blotting

As in Example 1, the phosphorylated GST-poly (Glu,Tyr) fused protein contained in samples for SDS "i (+) " to "vii (+) " and samples for SDS "i (-) " to "vii (-) " was detected by Western blotting.

### 4. Results

Fig. 4 is a fluorescent photograph showing results of Western blotting. In the figure, "i" shows results in the case of use of PDGFR-β as a tyrosine kinase, "ii" shows results in the case of use of VEGFR1, "iii" shows results in the case of use of VEGFR2, "iv" shows results in the case of use of HER1, "v" shows the results in the case of use of HER2, "vi" shows results in the case of use of HER4, and "vii" shows results in the case of used of IGF1R. In each of photographs of "i" to "vii", "-" is results obtained from a sample for SDS prepared using a substrate solution 4 containing no ATP. And, "+" is results obtained from a sample for SDS prepared using a substrate solution 3 containing ATP. P-ICD shows a position where an autophosphorylated tyrosine kinase appears, and P-GST-poly (Glu,Tyr) shows a position where a phosphorylated GST-poly (Glu,Tyr) fused protein appears.

In "+" of "i" to "vii", a single band was seen at positions where a phosphorylated GST-poly (Glu,Tyr) fused protein appears. Thereby, it was seen that the GST-poly (Glu,Tyr) fused protein prepared in Example 4 is phosphoerylated with various tyrosine kinases.

In "-" of "ii" to "iv", "vi" and "vii", a band is not seen at positions where a phosphorylated GST-poly (Glu,Tyr) fused protein appears because it is thought that ATP is not contained in a reaction solution in the enzyme reaction and the GST-poly (Glu,Tyr) fused protein appears is not phosphorylated. On the other hand, in - of "i" and "v", a very faint band was seen at positions where a phosphorylated GST-poly (Glu, Tyr) fused protein appears because it is thought that an antibody used in detection was non-specifically bound, or a small amount of ATP was mixed in a protein used in measurement.

### (Example 6) Detection of phosphorylation of fused protein by Western blotting when a receptor tyrosine kinase obtained from cell membrane is used

Herein, the GST-poly (Glu,Tyr) fused protein prepared in Example 4 was phosphorylated by using a receptor tyrosine kinase obtained from a cell membrane of a cultured cell derived from a breast cancer. A phosphorylated GST-poly (Glu,Tyr) fused protein was detected by Western blotting.

### 1. Method of preparing sample for reaction

A culturing cell (MDA-MB231) derived from a breast cancer was cultured to 80% confluent (about 107 cells) in a 225 cm² flask. This cultured cell and 1ml of a cell treating solution (containing 20 mM HEPES pH7.4, 0.2% PI, 10% glycerol, 200 µM Na₃VO₄, and 50 mM NaF) were mixed, and a cell membrane was destructed by pressurizing using a pestle to prepare a cell solution. The resulting cell solution was centrifuged, and the supernatant was discarded. A precipitate and a cell membrane solublizing solution (containing 20 mM HEPES pH7.4, 1% NP40, 0.2% PI, 10% glycerol, 200 µM Na₃VO₉, and 50 mM NaF) were mixed, a cell membrane was solubilized by pressurizing using a pestle, and this was centrifuged to recover the supernatant. This supernatant was used as a sample for a reaction in the following enzyme reaction. Similarly, a sample for a reaction solution was prepared from MDA-MB468 and SKBr3 which are a cultured cell derived from a breast cancer, respectively. Herein, a sample prepared from MDA-MB231 is designated as a sample for reaction "i", a sample prepared from MDA-MB468 is designated as a sample for reaction "ii", and a sample prepared from SKBr3 is designated as a sample for reaction "iii". All samples for a reaction were prepared so that a concentration of a contained protein became 0.8 mg/ml.

### 2. Enzyme reaction

25 µl of the sample for a reaction obtained by the aforementioned method and 25 µl of a substrate solution 5 (containing 20 mM HEPES pH7.4, 20 mM MnCl₂, 2 mM DTT, 1% NP40, 0.2% PI, 10% glycerol, 200 µM Na₃VO₄, 50 mM NaF, 100 µM ATP, and 5 µg of GST-poly (Glu,Tyr) fused protein) were mixed, followed by incubation at 25°C for 30 minutes. To this reaction solution was added 25 µl of the SDS sample buffer used in Example 1, and the mixture was boiled at 100°C for 5 minutes to stop the enzyme reaction. The thus prepared solution is designated as a sample for SDS "i". Similarly, sample for SDS "ii" and sample for SDS "iii" were prepared from the sample for reaction "ii" and the sample for reaction "iii".

### 3. Detection of phosphorylated fused protein by Western blotting

According to the same manner as that of Example 1, phosphorylated GST-poly (Glu, Tyr) fused proteins contained in the samples "i" to "iii" for SDS were detected by Western blotting.

### 4. Results

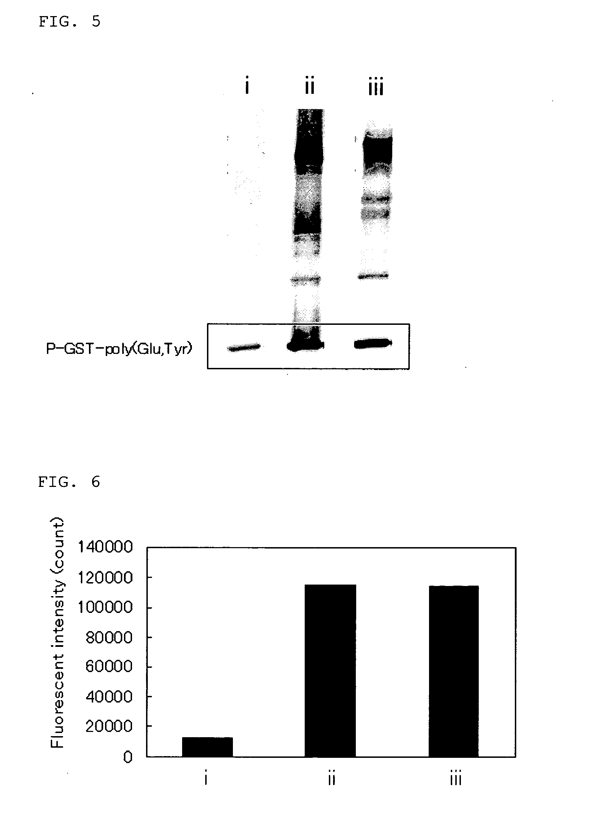

Fig. 5 is a fluorescent photograph showing results of Western blotting. In the figure, "i" indicates results in the case of use of a receptor tyrosine kinase extracted from a cell membrane of MDA-MB231, "ii" indicates results in the case of use of a receptor tyrosine kinase in which VEGFR1 is extracted from a cell membrane of MDA-MB468, and "iii" indicates results in the case of use of a receptor tyrosine kinase extracted from a cell membrane of SKBr3. P-GST-poly (Glu,Tyr) indicates a position where a phosphorylated GST-poly)(Glu,Tyr) fused protein appears.

In "i" to "iii", a single band was seen at a position where the phosphorylated GST-poly (Glu,Tyr) fused protein appears. Thereby, it was seen that a GST-poly (Glu,Tyr) fused protein is phosphorylated by a tyrosine kinase present in a cell membrane. In addition, since a GST-poly (Glu,Tyr) fused protein is phosphorylated by various tyrosine kinases as shown in Example 5, it is thought that a band detected herein is a GST-poly (Glu,Tyr) fused protein phosphorylated by various tyrosine kinases present in a cell membrane.

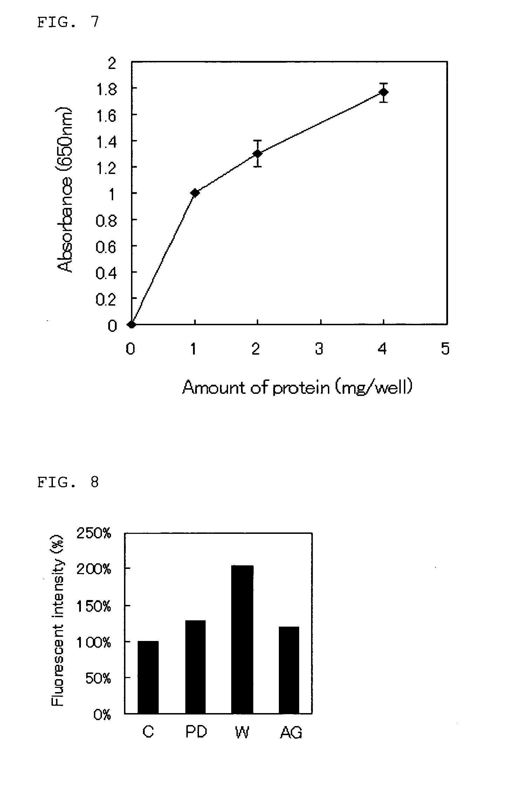

Fig. 6 is a graph showing results obtained by digitalizing a fluorescent intensity of a band of a phosphorylated GST-poly (Glu,Tyr) fused protein detected in a fluorescent photograph of Western blotting using an exclusive use analyzing software. In the figure, "i" indicates results in the case of use of a receptor tyrosine kinase extracted from a cell membrane of MDA-MB231, "ii" indicates results in the case of use of a receptor tyrosine kinase in which VEGFR1 is extracted from a cell membrane of MDA-MB468, and "iii" indicates results in the case of use of a receptor tyrosine kinase extracted from a cell membrane of SKBr3. An ordinate indicates a fluorescent intensity of a band.

When "i" to "iii" are compared, a great difference was seen in a fluorescent intensity between "i", and "ii" and "iii". This indicates that there is a great difference in an expression level and/or an enzyme activity level of a receptor tyrosine kinase between MDA-MB231, and MDA-MB468 and SKBr3. All of MDA-MB231, MDA-MB468 and SKBr3 are cultured cell derived from a breast cancer. From the foregoing, it is thought that it can be presumed that, in MDA-MB468 and SKBr3 showing a relatively great fluorescent intensity, abnormality of a receptor tyrosine kinase is one of causes for malignant alteration. On the other hand, it is thought that it can be presumed that, in MDA-MB231 showing a relatively small fluorescent intensity, abnormality of a receptor tyrosine kinase is not a cause for malignant alteration.

### (Example 7) Detection of phosphorylation of fused protein with ELISA when receptor tyrosine kinase obtained from cell membrane is used

Herein, using a receptor tyrosine kinase extracted from a cell membrane of a cultured cell derived from a breast cancer, the GST-poly (Glu, Tyr) fused protein prepared in Example 4 was phosphorylated. The phosphorylated GST-poly (Glu,Tyr) fused protein was detected with ELISA.

### 1. Method of preparing sample for reaction

A cultured cell (MDA-MB468) derived from a breast cancer was cultured to 80% confluent (about 107 cells) in a 225 cm² flask. This cultured cell and 1ml of the cell treating solution used in Example 6 were mixed, and a cell membrane was destructed by pressuring using a pestle to prepare a cell solution. The resulting cell solution was centrifuged, and the supernatant was discarded. A precipitate and the cell membrane solubilizing solution used in Example 6 were mixed, a cell membrane was solubilized by pressuring using a pestle, and this was centrifuged, and the supernatant was recovered. This supernatant was diluted with a cell membrane solubilizing solution to prepare samples for a reaction having a protein concentration of 0.02 g/ml, 0.04 g/ml and 0.08 g/ml, respectively. The thus prepared respective samples for a reaction, and a cell membrane solubilizing solution as a sample for a reaction containing no protein were used in the following enzyme reaction.

### 2. Binding of fused protein to plate for ELISA

As a plate for ELISA, a glutathione-coated plate (Reacti-Bind Clear Glutathione Coated Plates, 8-well Strip (PIERCE)) was used. First, each well of the plate was washed with TBS-T (containing 25 mM Tris, 150 mM NaCl and 0.05% Tween-20) three times. Then, 50 µl of the substrate solution 6 (TBS containing 5 µg/ml GST-poly (Glu,Tyr) fused protein) was place into each well, followed by incubation at 25°C for 1 hour while slightly shaking. After incubation, each well was washed with TBS-T two times, and further washed with 20 mM HEPES pH7.4 (containing 0.05% Tween-20) once. In such a way, the GST-poly (Glu, Tyr) fused protein was bound to a surface of the well of the plate for ELISA. This plate for ELISA was used in the following enzyme reaction.

### 3. Enzyme reaction, and detection of phosphorylated fused protein

50 µl of respective reaction solutions were placed into separate wells of the plate for ELISA, followed by incubation at 25°C for about 30 minutes. After incubation, 100 µl of a reaction stopping solution (TBS-T containing 1 mM EDTA) was added to each well, followed by further washing with TBS-T three times. Then, after each well was washed with 300 µl of StartingBlock T20 (TBS) Blocking Buffer (PIERCE), 100 µl of a primary antibody solution obtained by diluting a HRP-labeled primary antibody (p-Tys (PY20), sc-508 HRP (SANTA Cruz Biotechnology)) 1000 times with Blocking Buffer was placed into each well, and this was incubated at 25°C for about 1 hour and 30 minutes while slightly shaken. After incubation, each well was washed with TBS-T five times, 150 µl of a TMB solution (3,3',5,5'-Tetramethylbenzidine (TMB) Liquid Substrate System for ELISA (Sigma)) was placed into each well, a color was developed to an appropriate degree at room temperature for 5 to 30 minutes while light was shielded, and an absorbance (650nm) was measured with VersaMax (Molecular Device).

### 4. Results

Fig. 7 is a graph showing results of ELISA. In the figure, an ordinate indicates an absorbance (650nm), and an abscissa indicates an amount of protein (µg) per well (50 µl).

In Fig. 7, as an amount of protein became higher, that is, a tyrosine kinase amount became higher, a measured value was increased. Thereby, it was seen that a GST-poly (Glu,Tyr) fused protein is phosphorylated by a tyrosine kinase present in a cell membrane. In addition, since a GST-poly (Glu,Tyr) fused protein is phosphorylated by various tyrosine kinases as shown in Example 5, it is thought that a band detected herein is a GST-poly (Glu,Tyr) fused protein phosphorylated by various tyrosine kinases present in a cell membrane.

### (Example 8) Influence of inhibitor on kinase activity of receptor tyrosine kinase

Herein, using samples for a reaction (i to iii) obtained by the same method as that of Example 6, influence of various inhibitors on the kinase activity of a receptor tyrosine kinase was investigated.

### 1. Method of preparing sample for reaction

Samples for a reaction ("i" to "iii") obtained by the same method as that of Example 6 were used.

### 2. Enzyme reaction

Each 25 µof samples for a reaction were accommodated in four tubes. Among four tubes, to one tube was added 25 µl of a reaction solution (containing 20 mM HEPES pH7.4, 20 mM MnCl₂, 2 mM DTT, 0.1% NP-40, 0.2% PI, 10% glycerol, 100 µM Na₃VO₄, 100 µM ATP, and 5 µg of GST-poly (Glu,Tyr) fused protein) containing 100 µM PD158780 (Carbiochem) which is an inhibitor for HER1 and HER2, to other one tube was added 25 µl of a reaction solution containing 100 µM W4557 (Carbiochem) which is an inhibitor for HER1 and HER2, to other one tube was added 25 µl of a reaction solution containing 100 µM AG1478 (Carbiochem) which is an inhibitor for HER1 and HER2, and to a remaining one tube was added 25 µl of a reaction solution containing no inhibitor, followed by incubation at 25°C for 30 minutes. A reaction solution containing PD158780 is designated as "PD", a reaction solution containing W4557 is designated as "W", a reaction solution containing AG1478 is designated as "AG", and a reaction solution containing no inhibitor is designated as "C".

### 3. Detection of phosphorylated fused protein by Western blotting

Using these "PD", "W", "AG" and "C", Western blotting was performed according to the same method as that of Example 6. Results are shown by a graph in which a fluorescent intensity of a band of a phosphorylated GST-poly (Glu,Tyr) fused protein detected in a fluorescent photograph of Western blotting was digitalized using an exclusive use analyzing software.

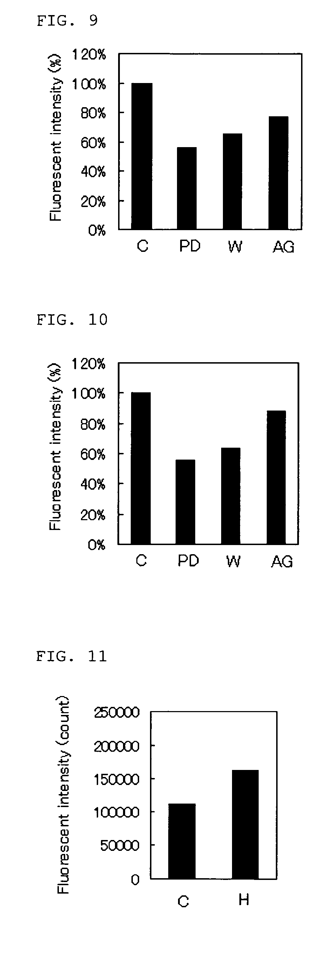

Fig. 8 is results obtained by using "PD", "W", "AG" and "C" prepared using a sample for a reaction "i", and Fig. 9 is results obtained by using "PD", "W", "AG" and "C" prepared using a sample for a reaction "ii". Fig. 10 is results obtained by using "PD", "W", "AG" and "C" prepared using a sample for a reaction "iii". An ordinate indicates a fluorescent intensity of each band as a value, letting a fluorescent intensity of a band when a reaction solution "C" containing no inhibitor is used, to be 100%.

### 4. Results

In Figs. 9 and 10, a fluorescent intensity of "PD", "W" and "AG" was reduced as compared with "C", because it is thought that the enzyme activity of a part of a receptor tyrosine kinase present in a cell membrane of MDA-MB468 and SKBr3 was inhibited with respective inhibitors of PD158780, W4557 and AG1478. On the other hand, in Fig. 8, reduction in a fluorescent intensity of "PD", "W" and "AG" was not seen, because it is thought that the enzyme activity of a receptor tyrosine kinase present in a cell membrane of MDA-MB231 was not inhibited with respective inhibitors of PD158780, W4557 and AG1478. From the above results, it was seen that the inhibiting ability of an inhibitor on the enzyme activity of a receptor kinase can be presumed using the measuring method of the present Example.

### (Example 9) Influence of ligand on kinase activity of receptor tyrosine kinase

Herein, using a sample for a reaction "iii" obtained by the same method as that of Example 6, influence of ligands for HER3 and HER4 on the kinase activity of a receptor tyrosine kinase was investigated.

### 1. Method of preparing sample for reaction

A sample for a reaction "iii" obtained by the same method as that of Example 6 was used.

### 2. Enzyme reaction

Each 25 µl of the sample for a reaction "iii" was accommodated in two tubes. Among them, to one tube was added 25 µl of a reaction solution (containing 20 mM HEPES pH7.4, 20 mM MnCl₂, 2 mM DTT, 0.1% NP-40, 0.2% PI, 10% glycerol, 100 µM Na₃VO₄, 100 µM ATP, and 5 µg of GST-poly (Glu,Tyr) fused protein) containing 100ng of Heregulin (Sigma) which is a ligand for HER3, and to other one was added 25 µl of a reaction solution containing no ligand, followed by incubation at 25°C for 30 minutes. A reaction solution containing Heregulin is designated as "H", and a reaction solution containing no Heregulin is designated as "C".

### 3. Detection of phosphorylated fused protein with Western blotting

Using these "H" and "C", Western blotting was performed according to the same method as that of Example 6. And, a fluorescent intensity of a band of a phosphorylated GST-poly (Glu,Tyr) fused protein detected in a fluorescent photograph was digitalized using an exclusive use analyzing software.

Fig. 11 is results obtained by using "H" and "C" prepared using the sample for a reaction "iii". An ordinate indicates a fluorescent intensity of a band.

### 4. Results

In Fig. 11, a fluorescent intensity of "H" was higher as compared with "C", because it is thought that a receptor tyrosine kinase present in a cell membrane of SKBr3 was activated with Heregulin. Hergulin is a ligand for HER3 and HER4. It is known that, when Heregulin binds to an extracellular domain of HER3, dimerization between HER3 and HER other than HER3 (HER1, HER3 or HER4) is induced, thereby, the tyrosine kinase activity is exhibited. In addition, it is known that binding of Heregulin to an extracellular domain of HER4 activates HER4. It is known that, while expression of HER2 and HER3 is strongly seen in SKBr3, HER1 and HER4 are expressed at an extremely small amount. From the forgoing, it is thought that increase in a fluorescent intensity in Fig. 11 is generated due to binding of Heregulin to an extracellular domain of HER3 and formation of a complex of HER3 and HSER2.

In addition, it is known that a tyrosine kinase site of HER3 has no phosphorylation activity. Therefore, it is thought that HER3 alone does not exhibit the activity of the tyrosine kinase and exhibits the activity of a tyrosine kinase after formation of a dimer with HER1, HER2 or HER4. Since activation of a receptor tyrosine kinase with Heregulin was seen in Fig. 11, it is thought that a dimer of HER2 and HER3 was formed. Thereby, it is thought that, in the measuring method of the present Example, a receptor kinase is obtained in the state where the function of forming a steric structure which can be formed upon activation is retained.

## Claims

1. A method for measuring the activity of a receptor kinase present in a cell membrane of a cell, comprising steps of:
preparing a sample containing the receptor kinase by separating a cytoplasm from the cell;
contacting the receptor kinase in the sample with a substrate to phosphorylate the substrate by the activity of the receptor kinase;
binding a labeling substance to the phosphorylated substrate; and
measuring the activity of the receptor kinase based on a result of detection of the labeling substance bound to the phosphorylated substrate.

2. The method according to claim 1, in the preparing step,
fragmentating the cell in a buffer solution;
mixing the buffer solution containing the fragmentated cell with a surfactant; and
preparing the sample by collecting the supernatant of the mixture.

3. The method according to claim 2, wherein the buffer solution has a pH of 4.0 to 9.0.

4. The method according to claim 2, wherein the surfactant is a nonionic surfactant which does not substantially bind to the receptor kinase.

5. The method according to any one of claims 1 to 4, wherein the substrate has an affinity tag, and the method further comprises steps of:
contacting the substrate having the affinity tag with a solid phase having a binding substance capable of binding to the affinity tag to form a complex;
obtaining the complex; and
obtaining the substrate from the complex by dissociating binding of the binding substance and the affinity tag.

6. The method according to any one of claims 1 to 5, wherein the labeling substance binds to the phosphorylated substrate through a primary antibody capable of binding to the phosphorylated substrate and a secondary antibody capable of binding to the primary antibody.

7. The method according to any one of claims 1 to 6, wherein the receptor kinase is a tyrosine kinase.

8. The method according to claim 7, wherein the tyrosine kinase is at least one selected from the group consisting of insulin-like growth factor receptor (IGFR), platelet-derived growth factor receptor (PDGFR), human epithelial growth factor receptor (HER) and vascular endothelial growth factor (VEGFR) .

9. The method according to claim 7 or 8, wherein the substrate is a substrate corresponding to a receptor tyrosine kinase.

10. The method according to claim 9, wherein the substrate is at least one selected from the group consisting of growth factor receptor-binding protein 2 (Grb2), myelin basic protein (MBP), histone H2B (HH2B), and phospholipase C gamma.

11. The method according to claim 9, wherein the substrate is a substrate which can be phosphorylated with a plurality of kinds of receptor tyrosine kinases.

12. The method according to claim 11, wherein the substrate comprises a peptide having an amino acid sequence including a glutamic acid residue and a tyrosine residue.

13. The method according to claim 12, wherein the amino acid sequence is at least one selected from the group consisting of:
an amino acid sequence A consisting of a glutamic acid residue and a tyrosine residue, in which a ratio of a glutamic acid residue and a tyrosine residue is 4:1,
an amino acid sequence B consisting of a glutamic acid residue and a tyrosine residue, in which a ratio of a glutamic acid residue and a tyrosine residue is 1:1,
an amino acid sequence C consisting of a glutamic acid residue, a tyrosine residue and an alanine residue, in which a ratio of a glutamic acid residue, an alanine residue and a tyrosine residue is 6:1:3,
an amino acid sequence D consisting of a glutamic acid residue, a tyrosine residue and an alanine residue, in which a ratio of a glutamic acid residue, an alanine residue and a tyrosine residue is 1:1:1, and
an amino acid sequence E consisting of a glutamic acid residue, a tyrosine residue, an alanine residue, and a lysine residue in which a ratio of a glutamic acid residue, a tyrosine residue, an alanine residue, and a lysine residue is 2:1:6:5.

14. The method according to any one of claims 1 to 13, wherein the labeling substance is a fluorescence substance or an enzyme.

15. A method for estimating influence of a receptor kinase inhibitor on a cell, comprising steps of:
measuring the activity of a receptor kinase by the method according to claim 1 to obtain a first measurement result;
measuring the activity of the receptor kinase contacted with the inhibitor by the method according to claim 1 to obtain a second measurement result; and
estimating influence of the inhibitor on the cell based on the first and second measurement results.

16. A method for estimating influence of a receptor kinase ligand on a cell, comprising steps of:
measuring the activity of a receptor kinase by the method according to claim 1 to obtain a first measurement result;
measuring the activity of the receptor kinase contacted with the ligand by the method according to claim 1 to obtain a second measurement result; and
estimating influence of the ligand on the cell based on the first and second measurement results.

17. A reagent kit for measuring the activity of a receptor kinase present in a cell membrane of a cell, comprising:
a substrate for the receptor kinase;
a phosphate group donor containing a phosphate group which is capable of being introduced into the substrate by the activity of the receptor kinase; and
a labeling substance capable of binding to the substrate with a phosphate group introduced therein.

18. The reagent kit according to claim 17, comprising:
a buffer solution having a pH of 4.0 to 9.0, which is added for fragmentating the cell; and
a surfactant capable of solibilizing the cell membrane.

19. The reagent kit according to claim 17 or 18, wherein the phosphate group donor is ATP or ADP.

20. The reagent kit according to any one of claims 17 to 19, wherein the labeling substance is a fluorescence substance or an enzyme.
